# EUROPÄISCHE PATENTSCHRIFT

(11) **EP 0 564 979 B1**
(45) Veröffentlichungstag und Bekanntmachung des Hinweises auf die Patenterteilung: **28.08.1996**
(21) Anmeldenummer: 93105419.1
(22) Anmeldetag: 01.04.1993
(51) Int. Cl.: C07C 15/16, C07C 2/86

(54) **Verfahren zur Herstellung von Dixylylpropan**
Method for the production of dixylylpropane
Procédé pour la fabrication de dixylylpropane

(30) Priorität: 10.04.1992 DE 4212055
(43) Veröffentlichungstag der Anmeldung: 13.10.1993
(73) Patentinhaber: HOECHST AKTIENGESELLSCHAFT, 65926 Frankfurt am Main (DE)
(72) Erfinder: Röhrscheid, Freimund, Dr., W-6233 Kelkheim/Ts. (DE)

(56) Entgegenhaltungen:
- CHEMICAL ABSTRACTS, Band 70, Nr. 17, 28. April 1969, S. 250, Zusammenfassung Nr. 76985r, Columbus, Ohio, US; R.N. VOLKOV et al: "Alkylation of o-xylene with propylene and isopropyl alcohol"
- HOUBEN-WEYL, Methoden der organischen Chemie, Band V/1b, 1972 , Georg Thieme Verlag, Stuttgart, DE, S. 376-382

## Beschreibung

Die Erfindung betrifft ein Verfahren zur Herstellung von Dixylylpropan.

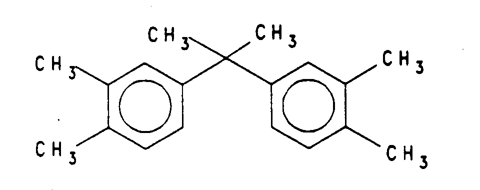

Dixylylpropan, auch als 2,2-Bis-(3,4-dimethylphenyl)-propan bezeichnet, ist beispielsweise ein Vorprodukt für die Herstellung Von Isopropyliden-bis-phthalsäureanhydrid. Letztere Verbindung ist ein Baustein für thermoplastisch verarbeitbare Polyimide. Die Herstellung von Dixylylpropan aus o-Xylol und 2,2-Dichlorpropan ist bereits beschrieben (US-A 2,712,543). Das benötigte 2,2-Dichlorpropan ist jedoch nur sehr schwer zugänglich.

Es stellte sich daher die Aufgabe, eine neue Synthese zu finden, die von leicht zugänglichen Chemikalien ausgeht und die den gewünschten Stoff in hoher Ausbeute liefert.

Die Erfindung bezieht sich daher auf ein Verfahren zur Herstellung von Dixylylpropan aus o-Xylol und Propen, bei dem stufenweise
a) o-Xylol mit Propen zu 1,2-Dimethyl-4-isopropylbenzol umgesetzt,
b) 1,2-Dimethyl-4-isopropylbenzol zu 1,2-Dimethyl-4-isopropenylbenzol katalytisch dehydriert,
c) 1,2-Dimethyl-4-isopropenylbenzol mit Chlorwasserstoff zu 1,2-Dimethyl-4-(α-chlor-isopropyl)-benzol umgesetzt und schließlich
d) 1,2-Dimethyl-4-(α-chlor-isopropyl)-benzol mit o-Xylol zum Dixylylpropan kondensiert wird.

Das Verfahren läßt sich schematisch wie folgt darstellen:

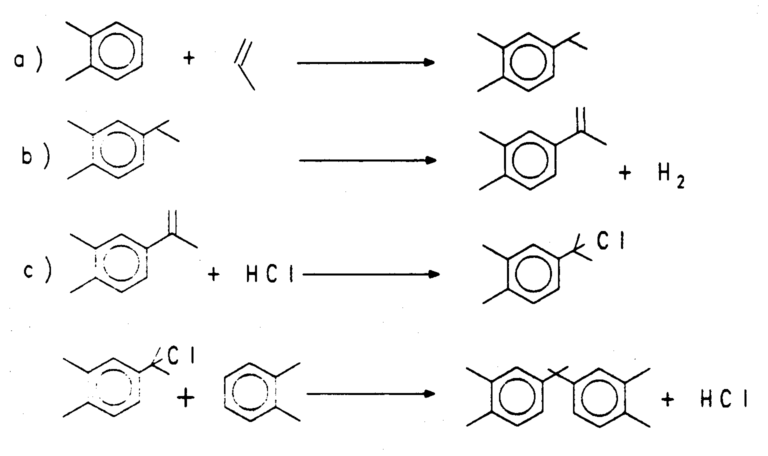

Das beanspruchte Verfahren zur Herstellung von Dixylylpropan benutzt die gut zugänglichen Verbindungen o-Xylol und Propen als Ausgangsverbindungen, liefert die gewünschte Verbindung in hoher Ausbeute und Isomeren-Reinheit und zeichnet sich durch einen geringen Anfall an Nebenprodukten aus. Sie ist somit für eine problemlose Weiterverarbeitung bestens geeignet.

Die Verfahrensweisen der einzelnen Stufen sind an sich bekannt. So erfolgt die Isopropylierung des o-Xylols in Stufe a) durch Einleiten von Propen in eine Mischung aus o-Xylol in Gegenwart eines Katalysators. Dabei soll der Katalysator nur mild alkylierend wirken und darf auch bei höherer Temperatur weder die Methylgruppen umlagern noch selbst mit der aromatischen Komponente reagieren.

In der Literatur wurden bisher zwei Typen von Katalysatoren für die Reaktion der Stufe a) beschrieben, die diesen Forderungen genügen. Ein Katalysator dieser Art ist BF₃-H₃PO₄ (A.V. Topchiev, et al., Dokl. Akad. Nauk SSSR, Vol. 134 (1960) S. 844-847, engl. Übersetzung: pp. 1101-1104). Bei seiner Anwendung liegen nach der Isopropylierung von o-Xylol im Zweiphasengemisch o-Xylol, 1,2-Dimethyl-3-isopropyl-benzol, 1,2-Dimethyl-4-isopropyl-benzol, Diisopropylxylole und Schwersieder in der organischen Phase vor.

Die besten Ausbeuten an 1,2-Dimethyl-4-isopropylbenzol können nur durch eine begrenzte Isopropylierung des o-Xylols erreicht werden, d.h. es werden keine stöchiometrischen Mengen der Ausgangsverbindungen eingesetzt. Die Reaktion mit begrenztem Umsatz führt somit, bezogen auf hergestelltes 1,2-Dimethyl-4-isopropyl-benzol, zu erhöhtem Katalysatorbedarf und erhöhtem Aufwand bei der Aufarbeitung.

Bekannt ist ferner die Reaktion von o-Xylol mit Propen in Gegenwart von AlCl₃-CH₃NO₂ als Katalysator (I.G. Gakh. et al., Z. Org. Khim, 1 [1965] S. 1626-1627; engl. Übersetzung: pp. 1646-1647). Als Hauptprodukt wird 1,2-Dimethyl-4-isopropylbenzol erhalten, daneben aber auch als Isomerisierungsprodukte 1,3-Dimethyl-5-isopropylbenzol und Trimethylbenzole. Nähere Angaben über die Durchführung der Versuche werden - bis auf die Reaktionstemperatur - nicht in der Veröffentlichung angegeben. Eine Nacharbeitung ergab erheblich abweichende Werte hinsichtlich Ausbeute und Zusammensetzung der erhaltenen Mischung.

Die Stufe a) des vorliegenden Verfahrens gemäß der Erfindung ermöglicht es, Propen mit o-Xylol nahe dem Molverhältnis 1:1 umzusetzen, wobei die eingesetzten Stoffe unter Verwendung der Katalysatoren BF₃·H₃PO₄ und AlCl₃·CH₃NO₂ fast vollständig in 1,2-Dimethyl-4-isopropylbenzol umgewandelt werden. Propen und o-Xylol werden im Molverhältnis 0,8-2,0, vorzugsweise 0,8-1,0, insbesondere 0,95-0,98:1 eingesetzt.

Vorzugsweise wird als Katalysator AlCl₃·CH₃NO₂ benutzt, der besonders gut aus dem Reaktionsgemisch entfernt werden kann. Der Katalysatorbestandteil AlCl₃ kann im Verhältnis von 0,001-0,3 Mol, vorzugsweise 0,003-0,05 Mol, insbesondere 0,005-0,02 Mol, pro Mol o-Xylol eingesetzt werden. Der molare Anteil von CH₃NO₂ kann von 1 bis 10, vorzugsweise 2 bis 4 pro Mol AlCl₃ betragen.

Durch eine geringe Chlorwasserstoffmenge wird die Aktivität des Katalysators erhöht.

Es ist von Vorteil, daß der Katalysator AlCl₃·CH₃NO₂ ohne Wäsche mit Wasser oder einem anderen Medium aus der Reaktionslösung durch Fällung und Abfiltrieren entfernt werden kann. Die Fällung erfolgt durch Zugabe von feinverteiltem Alkali- oder Erdalkaliacetaten, vorzugsweise durch die Acetate des Natriums, Kaliums und Calziums.

Die Umsetzung von o-Xylol mit Propen in Gegenwart der genannten Katalysatoren wird bei Temperaturen von 60-130°C, vorzugsweise bei 80-100°C innerhalb 2-10, vorzugsweise 3-8, insbesondere 4-6 Stunden durchgeführt. Eine Variante des Verfahrens besteht darin, die Anlagerung des Propens an das o-Xylol bei niedrigen Temperaturen von 0-60°C, vorzugsweise 0-40°C im ersten Schritt durchzuführen und anschließend den Reaktionsansatz auf die vorstehend genannten Reaktionstemperaturen zu erhöhen. Bei diesen Verfahren in zwei Schritten werden die neben dem 1,2-Dimethyl-4-isopropylbenzol gebildeten Verbindungen 1,2-Dimethyl-3-isopropylbenzol, 1,2-Dimethyl-3,5-diisopropylbenzol und 1,2-Dimethyl-3,4,6-triisopropylbenzol fast vollständig zu dem gewünschten 1,2-Dimethyl-4-isopropylbenzol komproportioniert und isomerisiert.

Besonders interessant bei dem Verfahren gemäß der Stufe a) ist es, daß alle organischen Komponenten wiederverwertet werden können. So kann beispielsweise das bei der destillativen Aufbereitung des Reaktionsansatzes anfallende Nitromethan sowie o-Xylol und der Destillationsrückstand in das Reaktionsgefäß überführt und dort nach Zugabe von weiteren Komponenten erneut der Reaktion zur Herstellung von 1,2-Dimethyl-4-isopropylbenzol unterworfen werden.

Die Dehydrierung des 1,2-Dimethyl-4-isopropylbenzols in Stufe b) erfolgt nach einer der bekannten Methoden, wie sie z.B. in Houben/Weyl "Methoden der organischen Chemie", Bd. 5/1b, S. 376 ff oder in US-A 3,429,941 beschrieben werden. Günstig ist es, einen Katalysator der Zusammensetzung Fe₂O₃-KOH-Cr₂O₃ zu verwenden.

Bei der Dehydrierung entsteht ein Gemisch aus 1,2-Dimethyl-4-isopropylbenzol und 1,2-Dimethyl-4-isopropenylbenzol, deren Siedepunkt nahe beieinander liegen. Dadurch gestaltet sich eine destillative Trennung bei den erforderlichen niedrigen Temperaturen (< 90°C) und dem dazugehörigen niedrigen Druck schwierig. Vorzugsweise wird deswegen das genannte Gemisch der weiteren Umsetzung unterworfen.

Hierzu werden in Stufe c) die leichtsiedenden Anteile vom Dehydrierungsprodukt abdestilliert und das Gemisch aus 1,2-Dimethyl-4-isopropylbenzol und 1,2-Dimethyl-4-isopropenylbenzol bei -30 bis +40°C, vorzugsweise bei -10 bis +10°C mit trockenem Chlorwasserstoff in ein Gemisch aus 1,2-Dimethyl-4-isopropylbenzol und 1,2-Dimethyl-4-(α-chlor-isopropyl)-benzol überführt. Aus diesem Gemisch kann unter vermindertem Druck das 1,2-Dimethyl-4-isopropylbenzol abdestilliert werden (44°C/2 mbar, 31°C/1 mbar). Das 1,2-Dimethyl-4-(α-chlor-isopropyl)-benzol wird in die Kondensationsreaktion der Stufe d) eingesetzt.

In der Stufe d) erfolgt die Synthese des 2,2-Dixylylpropans durch Umsetzung von 1,2-Dimethyl-4-(α-chlor-isopropyl)-benzol und o-Xylol durch Friedel-Crafts-Katalyse mit Aluminiumchlorid bei Temperaturen von -25 bis +30°C, vorzugsweise bei - 20° bis 0°C. Besonders bevorzugt ist die Umsetzung des in der Stufe c) anfallenden Gemisches aus 1,2-Dimethyl-4-(α-chlor-isopropyl)-benzol und 1,2-Dimethyl-4-isopropylbenzol mit o-Xylol, wodurch ein leicht trennbares Gemisch aus Nitromethan, o-Xylol, 1,2-Dimethyl-4-isopropylbenzol und Dixylylpropan entsteht. Die Aufarbeitung der Reaktionslösung erfolgt in einem nichtwäßrigen Medium analog der Stufe a).

Durch die wasserfreie Aufarbeitung wird die Friedel-Crafts-Synthese zu einem kostengünstigen Verfahren ohne aufwendige Wäschen und Phasentrennungen. Aus der Reaktionsmischung wird nach der Kondensation zum Dixylylpropan der restliche Chlorwasserstoff mit N₂ ausgetragen und in H₂O absorbiert. Die entgaste Reaktionslösung wird mit Alkali- oder Erdalkaliacetaten, vorzugsweise Calciumacetat, besonders bevorzugt Ca(OAc)₂/Ca(OH)₂ versetzt. Ausgefallenes Al(OAc)₂ und CaCl₂ werden abfiltriert.

Das Nitromethan, das bei einer Wasserwäsche ausgetragen wird, kann bei der durchgeführten wasserfreien Aufarbeitung durch Destillation zurückgewonnen werden, was die wirtschaftliche Seite des Verfahrens kennzeichnet. Außerdem findet keine Veruneinigung des Wassers durch CH₃NO₂ statt.

### Beispiele

### 1a) Isopropylierung von o-Xylol (Temperaturbedingungen gemäß Stand der Technik)

In einem 10-l-Kolben mit Rührer und Gaseinleitrohr wurden 6,37 kg o-Xylol und 0,165 kg Nitromethan eingefüllt. Unter Rühren wurden bei 20°C 0,12 kg wasserfreies Aluminiumchlorid zugegeben. In die gelbe Lösung wurden zuerst 5 g Chlorwasserstoff und dann im Laufe von zwei Stunden 2,02 kg Propen eingeleitet. Die Temperatur wurde durch Kühlung auf 40°C gehalten.
Am Ende der Umsetzung hatte die Reaktionsmischung die folgende Zusammensetzung: 1,4 % Nitromethan, 29,4 % o-Xylol, 23,0 % 1,2-Dimethyl-4-isopropylbenzol, 14,7 % 1,2-Dimethyl-3-isopropylbenzol, 24,5 % 1,2-Dimethyl-3,5-diisopropylbenzol, 8,3 % 1,2-Dimethyl-3,4,6-triisopropylbenzol.

### 1b) Komproportionierung und Isomerisierung zum 1,2-Dimethyl-4-isopropylbenzol

Der Ansatz nach Beispiel 1a) wurde auf 90°C geheizt und 8 Stunden bei dieser Temperatur gehalten. Die Reaktionsmischung hatte am Ende die folgende Zusammensetzung: 1,4 % Nitromethan, 15,9 % o-Xylol, 77,2 % 1,2-Dimethyl-4-isopropylbenzol, 1,8 % 1,2-Dimethyl-3-isopropylbenzol, 3,5 % 1,2-Dimethyl-3,5-diisopropylbenzol, 0,2 % Rest, nicht bestimmt.

### 1c) Fällung des Katalysators

In den Ansatz nach Beispiel 1b) wurde noch in heißem Zustand 0,25 kg Calziumacetat x 1/2 H₂O eingetragen, 2 Stunden bei 90°C gerührt, dann abgekühlt und die Suspension über eine Nutsche abgesaugt. Der Filterkuchen wurde zweimal mit je 0,15 kg o-Xylol gewaschen.
Es fallen ca. 8,3 kg Filtrat und 0,37 kg Metallsalze (trocken) an. Das Filtrat enthielt keine Chlorionen.

### 1d) Destillation des Filtrats von Beispiel 1c)

Das Filtrat konnte ohne weitere Vorbehandlung sofort an einer Kolonne destilliert werden.
Bei 1 bar wurden zuerst 0,98 kg Gemisch aus Nitromethan (11 %) und o-Xylol abdestilliert. Dann folgte eine fraktionierte Destillation bei 300 mbar. Nach einem Vorlauf von 0,12 kg einer Mischung aus o-Xylol und 1,2-Dimethyl-4-isopropylbenzol wurden 6,05 kg 1,2-Dimethyl-4-isopropylbenzol bei 155 ± 0,5°C erhalten.
Der Destillationsrückstand (1,16 kg) bestand aus 38 % (0,44 kg) 1,2-Dimethyl-4-isopropylbenzol, 23 % (0,27 kg) 1,2-Dimethyl-3-isopropylbenzol, 39 % (0,45 kg) 1,2-Dimethyl-3,5-diisopropylbenzol. Ausbeute: 6,05 kg 1,2-Dimethyl-4-isopropylbenzol = 85,03 % d. Th. bezogen auf eingesetztes Propen gemäß Beispiel 1a).

### 2) Recyklisierung der Nebenprodukte aus Beispiel 1d)

Im Reaktionskolben gemäß Beispiel 1 wurden vorgelegt: 0,980 kg o-Xylol/Nitromethan (89:11), 1,160 kg Destillationsrückstand und 0,120 kg Gemisch aus o-Xylol/1,2-Dimethyl-4-isopropylbenzol (64:36 %). Dem Ansatz wurden zusätzlich 4,220 kg o-Xylol, 0,057 kg Nitromethan und 0,120 kg AlCl₃ zugegeben, so daß sich die Mischung zusammensetzte aus: 5,167 kg o-Xylol, 0,165 kg Nitromethan, 0,120 kg AlCl₃, 0,483 kg 1,2-Dimethyl-4-isopropylbenzol, 0,270 kg 1,2-Dimethyl-3-isopropylbenzol und 0,450 kg 1,2-Dimethyl-3,5-diisopropylbenzol.

Der Ansatz wurde gemäß Beispiel 1a) mit 1,95 kg Propen isopropyliert und wie oben beschrieben weiter verarbeitet. Nach Filtration lagen 8,3 kg einer Reaktionsmischung der folgenden Zusammensetzung vor: 1,6 % Nitromethan, 5,2 % o-Xylol, 81,1 % 1,2-Dimethyl-4-isopropylbenzol, 3,0 % 1,2-Dimethyl-3-isopropylbenzol, 9,1 % 1,2-Dimethyl-3,5-diisopropylbenzol.

### 3) Einstufige Isopropylierung von o-Xylol

Ein 1-l-Kolben (Rührer, Gaseinleitungsrohr) wurde mit 636 g o-Xylol und 16,5 g Nitromethan beschickt. Unter Rühren wurden bei 20°C 12 g wasserfreies Aluminiumchlorid zugegeben, anschließend 2 g Chlorwasserstoff und dann innerhalb 2 1/2 Stunden 202 g Propen eingeleitet, wobei die exotherme Reaktion auf eine Temperatur von 90°C gehalten wurde. Im Anschluß an die Einleitung des Propens wurde der Reaktionsansatz für weitere Zeit bei 90°C gehalten und die Mengen der enthaltenen Komponenten in bestimmten Zeitabständen ermittelt. Die Werte in Gew.-% sind in der Tabelle angeführt.

| | Gesamt-Reaktionsdauer (Stunden) | | | | |
|---|---|---|---|---|---|
| | 2 1/2 | 4 | 6 | 8 1/2 | 10 1/2 |
| Nitromethan | 1,7 | 1,3 | 1,3 | 1,3 | 1,5 |
| o-Xylol | 25,6 | 17,9 | 16,6 | 16,2 | 15,0 |
| 1,2-Dimethyl-4-isopropylbenzol | 44,7 | 74,2 | 76,9 | 78,3 | 78,5 |
| 1,2-Dimethyl-3-isopropylbenzol | 5,3 | < 1 | < 1 | < 1 | < 0,5 |
| 1,2-Dimethyl-3,5-diisopropylbenzol | 21,8 | 5,9 | 4,6 | 3,7 | 3,5 |
| 1,2-Dimethyl-3,4,6-triisopropylbenzol | 0,5 | 0 | 0 | 0 | 0 |

Die Fällung des Katalysators und die weitere Aufarbeitung wurde wie in den vorstehenden Beispielen durchgeführt.

### 4) Katalytische Dehydrierung von 1,2-Dimethyl-4-isopropylbenzol

1 kg 1,2-Dimethyl-4-isopropylbenzol und 3 kg Wasser wurden im Laufe einer Stunde in einen Verdampfer (ca. 300°C) gefördert und anschließend durch einen elektrisch beheizten Überhitzer auf 590°C aufgeheizt. Das Gemisch durchströmte dann einen auf 610°C beheizten Röhrenreaktor, der 3 l eines körnigen Katalysators [Typ "Shell 105", Zusammensetzung Fe₂0₃ (87 %) - KOH (11 %) - Cr₂O₃ (2 %), gemäß US-A 2 461 147] in dichter Schüttung enthielt. Die Kontaktzeit betrug 3,2 Sek.. Das Reaktionsgemisch wurde in einem Kondensator mit Wasser stark abgekühlt, in den gleichzeitig 40 g einer 0,5 %igen wäßrigen Lösung von 4-tert.Butylbrenzkatechin pro Stunde als Olefinstabilisator eingetropft wurde.
Nach Trennung der Phasen fielen 0,95 kg Rohprodukt an.
Die organische Phase wurde mit zusätzlichen 0,8 g 4-tert.-Butrylbrenzcatechin versetzt und bei 10 mbar fraktioniert destilliert, wobei 5,8 Gew.-% Vorlauf, 24,9 Gew.-% 1,2-Dimethyl-4-isopropylbenzol (Kp 72°C/10 mbar), 68,2 Gew.-% 1,2-Dimethyl-4-isopropenylbenzol (Kp 76°C/10 mbar) und 1,1 Gew.-% höher siedende Bestandteile anfielen.

### 5) Anlagerung von Chlorwasserstoff an 1,2-Dimethyl-4-isopropenylbenzol

Ein 0,25-l-Kolben mit wandgängigen Rührer und Gaseinleitrohr wurde mit Chlorwasserstoffgas gefüllt. Darauf wurden 73 g 1,2-Dimethyl-4-isopropenylbenzol in 30 Minuten unter kräftigem Rühren bei -5 bis -10°C zugetropft, wobei Chlorwasserstoff nachdosiert wurde. Die Gasaufnahme war fünf Minuten nach Ende des Zutropfens beendet. Vom Reaktionsprodukt wurde unter vermindertem Druck (35°C/0,8 mbar) Chlorwasserstoff abgezogen und 89,6 g (98,2 % d.Th.) 1,2-Dimethyl-4-(α-chlor-isopropyl)-benzol erhalten, Fp.: -20 bis -19°C, Zersetzung bei > 50°C/1 mbar unter HCl-Abspaltung.

| Elementaranalyse (C₁₁H₁₅Cl): | | | |
|---|---|---|---|
| gef.: | C 72,5 %; | H 8,15 %; | Cl 19,1 % |
| ber.: | C 72,31 %; | H 8,28 %; | Cl 19,41 % |

### 6) Herstellung von Dixylolpropan

### 6a) Kondensation von 1,2-Dimethyl-4-(α-chlor-isopropyl)-benzol mit o-Xylol

In einem 0,5-l-Kolben mit Rührer und Gaseinleitrohr wurden 106 g o-Xylol vorgelegt und bei 0°C mit Chlorwasserstoffgas gesättigt. Danach wurden 146 g 1,2-Dimethyl-4-isopropenylbenzol unter weiterem Einleiten von Chlorwasserstoffgas und kräftigem Rühren in 45 Min. zugetropft. Nach dem Zutropfen wurde noch weitere fünf Minuten Chlorwasserstoffgas eingeleitet (insgesamt ca. 38 g Chlorwasserstoff).

In einem 1-l-Kolben mit Rührer und Gaseinleitrohr wurden 424 g o-Xylol, 6,6 g AlCl₃ und 9,1 g CH₃NO₂ bei -5°C bis 0°C mit Chlorwasserstoffgas gesättigt. Danach wurde innerhalb 75 Min. die vorstehend beschriebene Lösung von 1,2-Dimethyl-4-(α-chlor-isopropyl)-benzol und o-Xylol unter kräftigem Rühren in das mit Chlorwasserstoffgas gesättigte o-Xylol eingetropft. Der Fortgang der Reaktion war an der kräftigen Gasentwicklung erkennbar.

Aus der Reaktionslösung wurde bei 0°C mit Stickstoff der gelöste Chlorwasserstoff vertrieben. Danach wurden 13,4 g feinverteiltes Calciumacetat x 1/2 H₂O unter Rühren zugefügt. Der ausgefällte Niederschlag wurde abgesaugt und mit 25 g o-Xylol gewaschen.

Die vereinigten Filtrate (705 g) wurden fraktioniert destilliert. Bei Normaldruck und 110-145°C wurden 360 g o-Xylol und Nitromethan erhalten. Das restliche o-Xylol (78 g) wurde bei 20 mbar bis zu einer Kopftemperatur von 45°C abdestilliert. Schließlich wurde Dixylylpropan bei 151°C/2,3 mbar destilliert. Ausbeute: 232 g (92,3 % d.Th.).

### 6b) Herstellung von Dixylolpropan aus einem Gemisch von 1,2-Dimethyl-4-isopropenylbenzol und 1,2-Dimethyl-4-isopropylbenzol

Ein Produktgemisch aus der Dehydrierung gemäß Beispiel 4 wurde destilliert. Dabei wurde ein Vorlauf abdestilliert und anschließend die Fraktion 72-76°C/10 mbar geschnitten. 196,8 g dieser Fraktion (Gehalt: 25,8 Gew.-% 1,2-Dimethyl-4-isopropylbenzol und 74,2 Gew.-% 1,2-Dimethyl-4-isopropenylbenzol) enthielten 146 g 1,2-Dimethyl-4-isopropenylbenzol. Diese Menge wurde bei 0°C mit 106 g o-Xylol vermischt, gemäß Beispiel 6a mit HCl umgesetzt und anschließend bei -5 bis 0°C kondensiert, wobei 424 g o-Xylol, 6,6 g AlCl₃ und 9,1 g CH₃NO₂ eingesetzt wurden. Nach der Katalysatorentfernung wurde fraktioniert destilliert. Dixylylpropan ließ sich sauber von o-Xylol und 1,2-Dimethyl-4-isopropylbenzol abtrennen. Ausbeute: 225,3 g (89,4 % d.Th.) Dixylylpropan.

## Patentansprüche

1. Verfahren zur Herstellung von Dixylylpropan aus o-Xylol und Propen, dadurch gekennzeichnet, daß
a) o-Xylol mit Propen zu 1,2-Dimethyl-4-isopropylbenzol umgesetzt,
b) 1,2-Dimethyl-4-isopropylbenzol zu 1,2-Dimethyl-4-isopropenylbenzol katalytisch dehydriert,
c) 1,2-Dimethyl-4-isopropenylbenzol mit Chlorwasserstoff zu 1,2-Dimethyl-4-(α-chlor-isopropyl)-benzol umgesetzt und schließlich
d) 1,2-Dimethyl-4-(α-chlor-isopropyl)-benzol mit o-Xylol zum Dixylylpropan kondensiert wird.

2. Verfahren nach Anspruch 1, dadurch gekennzeichnet, daß in Stufe a) ein Friedel-Crafts-Katalysator verwendet wird, vorzugsweise BF₃·H₃PO₄ oder AlCl₃·CH₃NO₂, insbesondere AlCl₃·CH₃NO₂.

3. Verfahren nach Anspruch 1 oder 2, dadurch gekennzeichnet daß die Umsetzung in Stufe a) bei 60-130°C, vorzugsweise 80-100°C durchgeführt wird.

4. Verfahren nach einem oder mehreren der Ansprüche 1 bis 3, dadurch gekennzeichnet daß Propen und o-Xylol im Mol-Verhältnis 0,8-2,0, vorzugsweise 0,8-1,0, insbesondere 0,95-0,98:1 eingesetzt werden.

5. Verfahren nach einem oder mehreren der Ansprüche 1 bis 4, dadurch gekennzeichnet, daß in Stufe a) AlCl₃ in Mengen von 0,001-0,3 Mol pro Mol o-Xylol eingesetzt wird.

6. Verfahren nach einem oder mehreren der Ansprüche 1 bis 5, dadurch gekennzeichnet, daß in Stufe a) das AlCl₃ des Katalysators AlCl₃·CH₃NO₂ nach der Reaktion durch Zugabe eines Alkali- oder Erdalkaliacetats gefällt und abfiltriert wird.

7. Verfahren nach einem oder mehreren der Ansprüche 1 bis 6, dadurch gekennzeichnet, daß das katalysatorfreie 1,2-Dimethyl-4-isopropylbenzol, erhalten nach Stufe a), fraktioniert destilliert wird und der Vorlauf sowie der Rückstand in den Reaktor der Stufe a) zur erneuten Umsetzung zurückgeführt werden.

8. Verfahren nach einem oder mehreren der Ansprüche 1 bis 7, dadurch gekennzeichnet, daß in Stufe b) ein Katalysator auf Basis Fe₂O₃·KOH·Cr₂O₃ verwendet wird.

9. Verfahren nach einem oder mehreren der Ansprüche 1 bis 8, dadurch gekennzeichnet, daß in Stufe c) Chlorwasserstoff bei -30 bis +40°C, vorzugsweise bei -10 bis +10°C angelagert wird.

10. Verfahren nach Anspruch 9, dadurch gekennzeichnet, daß ein Gemisch aus 1,2-Dimethyl-4-isopropylbenzol und 1,2-Dimethyl-4-isopropenylbenzol mit HCl umgesetzt wird.

11. Verfahren nach einem oder mehreren der Ansprüche 1 bis 10, dadurch gekennzeichnet, daß in Stufe d) ein Gemisch aus 1,2-Dimethyl-4-(α-chlor-isopropyl)-benzol und 1,2-Dimethyl-4-isopropylbenzol mit o-Xylol umgesetzt wird.

12. Verfahren nach Anspruch 11, dadurch gekennzeichnet, daß die Reaktion der Stufe d) bei -25 bis +30°C, vorzugsweise bei -20 bis 0°C durchgeführt wird.

## Claims

1. A process for the preparation of dixylylpropane from o-xylene and propene, which comprises
a) reacting o-xylene with propene to give 1,2-dimethyl-4-isopropylbenzene,
b) catalytically dehydrogenating 1,2-dimethyl-4-isopropylbenzene to give 1,2-dimethyl-4-isopropenylbenzene,
c) reacting 1,2-dimethyl-4-isopropenylbenzene with hydrogen chloride to give 1,2-dimethyl-4-(α-chloroisopropyl)benzene and finally
d) condensing 1,2-dimethyl-4-(α-chloroisopropyl)benzene with o-xylene to give dixylylpropane.

2. The process as claimed in claim 1, wherein a Friedel-Crafts catalyst is used in step a), preferably BF₃·H₃PO₄ or AlCl₃·CH₃NO₂, in particular AlCl₃·CH₃NO₂.

3. The process as claimed in claim 1 or 2, wherein the reaction in step a) is carried out at 60-130°C, preferably at 80-100°C.

4. The process as claimed in one or more of claims 1 to 3, wherein propene and o-xylene are employed in a molar ratio of 0.8-2.0, preferably 0.8-1.0, in particular 0.95-0.98:1.

5. The process as claimed in one or more of claims 1 to 4, wherein AlCl₃ is employed in step a) in amounts of 0.001-0.3 mol per mole of o-xylene.

6. The process as claimed in one or more of claims 1 to 5, wherein the AlCl₃ of the catalyst AlCl₃·CH₃NO₂ is precipitated after the reaction by addition of an alkali metal or alkaline earth metal acetate and filtered off in step a).

7. The process as claimed in one or more of claims 1 to 6, wherein the catalyst-free 1,2-dimethyl-4-isopropylbenzene, obtained by step a), is fractionally distilled and the forerun and the residue are recycled to the reactor of step a) for renewed reaction.

8. The process as claimed in one or more of claims 1 to 7, wherein a catalyst based on Fe₂O₃·KOH·Cr₂O₃ is used in step b).

9. The process as claimed in one or more of claims 1 to 8, wherein hydrogen chloride is added at -30 to +40°C, preferably at -10 to +10°C, in step c).

10. The process as claimed in claim 9, wherein a mixture of 1,2-dimethyl-4-isopropylbenzene and 1,2-dimethyl-4-isopropenylbenzene is reacted with HCl.

11. The process as claimed in one or more of claims 1 to 10, wherein a mixture of 1,2-dimethyl-4-(α-chloroisopropyl)benzene and 1,2-dimethyl-4-isopropylbenzene is reacted with o-xylene in step d).

12. The process as claimed in claim 11, wherein the reaction of step d) is carried out at -25 to +30°C, preferably at -20 to 0°C.

## Revendications

1. Procédé pour la préparation du dixylylpropane à partir de l'o-xylène et du propène caractérisé en ce que
a) on fait réagir l'o-xylène avec le propène pour obtenir le 1,2-diméthyl-4-isopropylbenzène,
b) on effectue une déshydrogènation catalytique du 1,2-diméthyl-4-isopropylbenzène en le 1,2-diméthyl-4-isopropénylbenzène,
c) on fait réagir le 1,2-diméthyl-4-isopropénylbenzène avec du chlorure d'hydrogène pour obtenir le 1,2-diméthyl-4-(α-chloro-isopropyl)-benzène, et finalement
d) on condense le 1,2-diméthyl-4-(α-chloro-isopropyl)-benzène avec l'o-xylène en le dixylylpropane.

2. Procédé selon la revendication 1, caractérisé en ce que à l'étape a) on utilise un catalyseur de Friedel-Crafts, de préférence BF₃.H₃PO₄ et AlCl₃.CH₃NO₂ plus particulièrement AlCl₃.CH₃NO₂.

3. Procédé selon la revendication 1 ou 2, caractérisé en ce qu'on met en oeuvre la réaction de l'étape a) de 60 à 130°C, de préférence de 80 à 100°C.

4. Procédé selon une ou plusieurs des revendications 1 à 3, caractérisé en ce qu'on utilise le propène et l'o-xylène dans un rapport molaire de 0,8 à 2,0, de préférence de 0,8 à 1,0, plus particulièrement de 0,95 à 0,98:1.

5. Procédé selon une ou plusieurs des revendications 1 à 4, caractérisé en ce que dans l'étape a) on utilise AlCl₃ dans des quantités de 0,001 à 0,3 mole par mole d'o-xylène.

6. Procédé selon une ou plusieurs des revendications 1 à 5, caractérisé en ce que dans l'étape a), on précipite après la réaction AlCl₃ du catalyseur AlCl₃.CH₃NO₂ par addition d'un acétate de métal alcalin ou alcalino-terreux, et on le sépare par filtration.

7. Procédé selon une ou plusieurs des revendications 1 à 6, caractérisé en ce qu'on distille par fractionnement le 1,2-diméthyl-4-isopropyl-benzène exempt catalyseur obtenu à l'étape a) et on recycle les produits de tête ainsi que le résidu dans le réacteur de l'étape a) pour une réaction ultérieure.

8. Procédé selon une ou plusieurs des revendications 1 à 7, caractérisé en ce qu'on utilise à l'étape b) un catalyseur à base de Fe₂O₃-KOH-Cr₂O₃.

9. Procédé selon une ou plusieurs des revendications 1 à 8, caractérisé en ce que à l'étape c), on obsorbe le chlorure d'hydrogène à une température d'au moins 30 à +40°C, de préférence à -10 à +10°C.

10. Procédé selon la revendication 9, caractérisé en ce qu'on fait réagir un mélange de 1,2-diméthyl-4-isopropylbenzène et de 1,2-diméthyl-4-iso-propénylbenzène avec HCl.

11. Procédé selon une ou plusieurs des revendications 1 à 10, caractérisé en ce que à l'étape d), on fait réagir un mélange de 1,2-diméthyl-4-(α-chloro-isopropyl)-benzène et de 1,2-diméthyl-4-isopropylbenzène avec l'o-xylène.

12. Procédé selon la revendication 11, caractérisé en ce que on met en oeuvre la réaction de l'étape d) à une température de -25 à +30°C, de préférence de -20 à 0°C.
